(19) Europäisches Patentamt
European Patent Office
Office européen des brevets

(11) Veröffentlichungsnummer : **0 329 599 B1**

(12) # EUROPÄISCHE PATENTSCHRIFT

(45) Veröffentlichungstag der Patentschrift :
04.12.91 Patentblatt 91/49

(51) Int. Cl.$^5$ : **A61M 1/00,** A61M 3/02,
A61B 1/12

(21) Anmeldenummer : 89730031.5

(22) Anmeldetag : 15.02.89

(54) Vorrichtung zur Perfusion von Körperhöhlen.

(30) Priorität : 17.02.88 DE 3805368

(43) Veröffentlichungstag der Anmeldung :
23.08.89 Patentblatt 89/34

(45) Bekanntmachung des Hinweises auf die
Patenterteilung :
04.12.91 Patentblatt 91/49

(84) Benannte Vertragsstaaten :
AT BE CH DE ES FR GB GR IT LI LU NL SE

(56) Entgegenhaltungen :
EP-A- 0 100 682
WO-A-86/01390

(56) Entgegenhaltungen :
DD-A- 124 498
DE-C- 3 338 758
US-A- 3 604 419
US-A- 4 180 074

(73) Patentinhaber : Wiest, Peter P., Dipl.-Ing.
Hessenallee 8
W-1000 Berlin 19 (DE)

(72) Erfinder : Wiest, Peter P., Dipl.-Ing.
Hessenallee 8
W-1000 Berlin 19 (DE)

(74) Vertreter : Lüke, Dierck-Wilm, Dipl.-Ing.
Gelfertstrasse 56
W-1000 Berlin 33 (DE)

EP 0 329 599 B1

## Beschreibung

Die Erfindung bezieht sich auf eine Vorrichtung zur Perfusion von Körperhöhlen gemäß den Merkmalen des Anspruches 1.

Eine Vorrichtung der gattungsgemäßen Art ist aus der WO-A 86/01390 vorbekannt. Diese Vorrichtung ist mit einer von einem Vorratsbehälter über eine Perfusionsleitung und ein medizinisches Instrument zugeführten Flüssigkeit, mit einer an die Perfusionsleitung angeschlossenen Pumpe, mit einem zwischen der Pumpe und dem medizinischen Instrument an die Perfusionsleitung angeschlossenen Druckaufnehmer, mit einer Vorrichtung zur Messung des Volumenstromes und mit einer an den Druckaufnehmer, die Pumpe und die Vorrichtung zur Messung des Volumenstromes angeschlossenen Regel-und Meßelektronik ausgestattet, die den aktuellen Druck in der Körperhöhle durch Auswertung des vom Druckaufnehmer gemessenen Druckes ermittelt. Nachteilig bei dieser bekannten Vorrichtung ist, daß keine genaue Messung des tatsächlichen Druckes während der Flüssigkeitszuführung möglich ist, da die zur Korrektur benutzten Parameter Höhen-Differenz, Querschnitt des medizinischen Instrumentes und Volumenstrom nicht ausreichen, um auf den tatsächlichen Druck in der Körperhöhle zurückzuschließen.

Es ist ferner aus der DE 3338758C2 eine weitere Vorrichtung zur Perfusion von Körperhöhlen mit einer Flüssigkeit vorbekannt. Der hierbei verwendete Druckaufnehmer ermittelt den IST-Wert des Druckes innerhalb der Perfusionsleitung, in welche der Druckaufnehmer eingesetzt ist. Eine genaue Messung des Druckes in der Körperhöhle selbst kann hierdurch nicht erfolgen, da Strömungs- und Druckverluste innerhalb des Perfusionsleitung und innerhalb des medizinischen Instrumentes den gemessenen Druckwert verfälschen.

Zur genauen Messung des Druckes innerhalb der Körperhöhle müßte daher der Druckaufnehmer am distalen Ende des medizinischen Instrumentes, insbesondere ein Endoskop, wie z.B. ein Arthroskop, angeordnet werden. Dies hat jedoch den Nachteil der großen mechanischen, thermischen und chemischen Belastung des Druckaufnehmers, insbesondere beim Sterilisationsvorgang des medizinischen Instrumentes. Auch ist ein dann erfoderlicher Miniatur-Druckaufnehmer relativ teuer. Darüber hinaus könnte ein zweites medizinisches Instrument nur zur Messung des aktuellen Druckes in der Körperhöhle eingesetzt werden. Dies würde jedoch den Patienten und auch die Handhabung der Vorrichtung zusätzlich belasten. Es würden zusätzliche Sterilisationmaßmahmen notwendig sein. Auch wäre die Meßunsicherheit sehr groß, weil eine unbemerkte Verstopfung oder Undichtigkeit des zusätzlichen medizinischen Instrumentes die Druckmessung erheblich verfälschen könnte.

Der Erfindung liegt von daher die Aufgabe zugrunde, eine Vorrichtung zur Perfusion von Körperhöhlen der gattungsgemäßen Art zu schaffen, wobei ein vorgewählter, genau bestimmbarer Druck in der Körperhöhle aufgebaut und aufrechterhalten und der Druck in der Körperhöhle genauestens gemessen werden kann.

Die Lösung dieser Aufgabe ergibt sich aus den kennzeichnenden Merkmalen des Anspruches. Erfindungsgemäß werden die Leitwerte der Perfusionsleitung und des Instrumentes über eine Kalibrierungsmessung ermittelt. Dies geschieht durch eine kurzzeitige Probeförderung der Flüssigkeit mittels der Pumpe durch das Instrument und durch den Perfusionsschlauch gegen den normalen Atmosphärendruck für verschiedene Volumenströme. Aus den Parametern Volumenstrom, gemessener Druck und äußerem Atmosphärendruck können die Leitwerte bzw. der gemeinsame Leitwert für die aktuell benutzte Schlauch-Instrumenten-Kombination ermittelt und in der Meß-und Regelelektronik abgespeichert werden. Bei einer derartigen Messung der Leitwerte wird natürlich die Höhe des medizinischen Instrumentes ebenso berücksichtigt und geht mit in den Leitwert ein. Über die derart ermittelten tatsächlichen Leitwerte wird der tatsächliche Druck in der Körperhöhle gemäß einer Formel genauestens zu jeder Zeit der Perfusion, sozusagen in Echtzeit, errechnet und angezeigt. Die Kenntnis des tatsächlichen Druckes in der Körperhöhle erlaubt weiterhin eine Regelung des tatsächlichen Soll-Druckes. Der Leitwert der Perfusionsleitung zwischen dem Druckaufnehmer und dem medizinischen Instrument sowie der Leitwert des Instrumentes selbst werden zur genauesten Ermittlung des aktuellen Druckes in der Körperhöhle verwendet, wodurch der Druck in der Körperhöhle durch kontinuierliche Regelung der Förderleistung der Pumpe unter Berücksichtigung des ermittelten Leitwertes genauestens gemessen und auf einem voreingestellten Wert konstant gehalten werden kann. Der Leitwert ist der Reziprokwert des Fließwiderstandes der Flüssigkeit innerhalb der Perfusionsleitung und innerhalb des medizinischen Instrumentes. Mit der erfindungsgemäßen Vorrichtung kann somit der aktuelle Druck in der Körperhöhle genauestens ohne Zuhilfenahme eines zweiten medizinischen Instrumentes ermittelt werden. Dies ermöglicht eine einfache Handhabung der Vorrichtung bei hoher Sicherheit und großer Meßzuverlässigkeit. Bei gleichem Leitwert der Kanüle des medizinischen Instrumentes kann mit größerem Volumenstrom gearbeitet werden, ohne durch den Druckabfall in der Kanüle des medizinischen Instrumentes mit Meßwertunsicherheiten bezüglich des Körperhöhlendruckes rechnen zu müssen. Auch ist eine Selbstüberwachung möglich, da bei einer bestimmten Förderleistung der Pumpe vom Druckaufnehmer mindestens der Strömungsdruck angezeigt werden muß. Bei Anzeige des apparativen Druckes in der Perfusionsleitung und des errechneten Druckes in der Körperhöhle kann der Leitwert

2

EP 0 329 599 B1

der Kanüle des medizinischen Instrumentes abgeschätzt werden.

Weitere vorteilhafte Ausgestaltungen der Erfindung ergeben sich aus den Unteransprüchen.

Die erfindungsgemäße Vorrichtung ist nachfolgend anhand eines in den Zeichnungen dargestellten Ausführungsbeispieles einer Vorrichtung zur Perfusion von Körperhöhlen näher erläutert. Es zeigen :

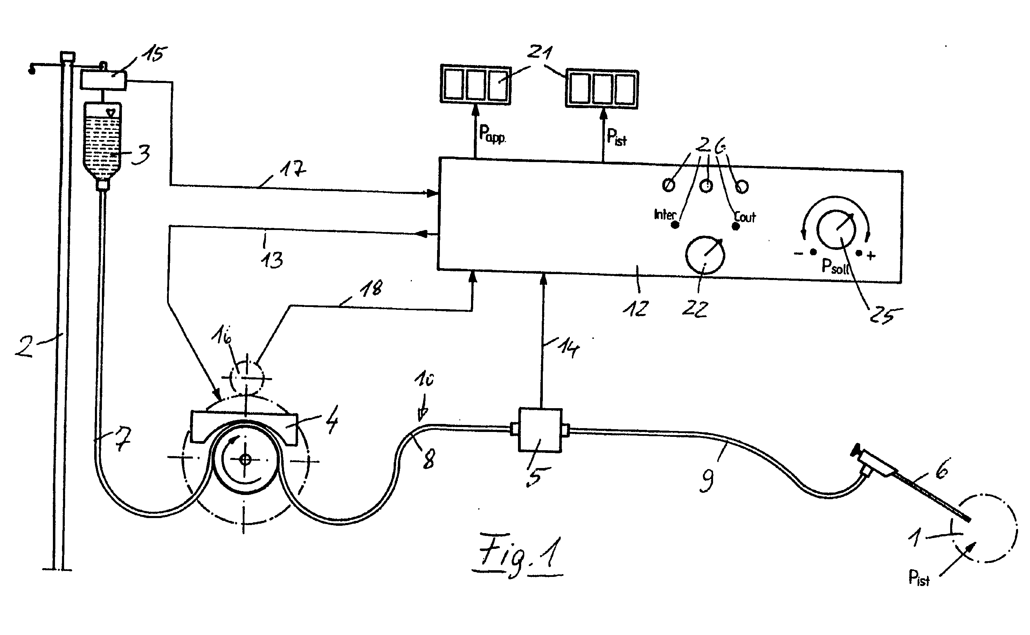

Fig. 1    eine Prinzipdarstellung der Vorrichtung,

Fig. 2    ein Blockschaltbild der Regel- und Meßeinheit der Vorrichtung und

Fig. 3    den Programmablaufplan der Regel- und Meßeinheit.

Die Vorrichtung zur Perfusion von Körperhöhlen 1 umfaßt einen an einem Stativ 2 aufgehängten Vorratsbehälter 3 für eine Perfusions-Flüssigkeit, eine Pumpe 4, die insbesondere als Peristaltikpumpe ausgebildet ist, einen Druckaufnehmer 5, ein medizinisches Instrument 6, insbesondere ein Endoskop, wie z.B. ein Arthroskop, eine Perfusionsleitung 10 aus Schlauchleitungen 7, 8 und 9 sowie eine Regel- und Meßelektronik 12, die über Verbindungsleitungen 13, 14 mit der Pumpe 4 bzw. dem Druckaufnehmer 5 zur Meßwertübertragung elektrisch verbunden ist. Ferner ist der Vorratsbehälter 3 am Stativ 2 über einen Gewichtssensor 15 aufgehängt. An der Pumpe 4 ist ein Drehzahlsensor 16 angebracht. Der Gewichtssensor 15 und der Drehzahlsensor 16 sind mittel Zuleitungen 17, 18 zur Meßwertübertragung mit der Regel- und Meßeinheit 12 verbunden.

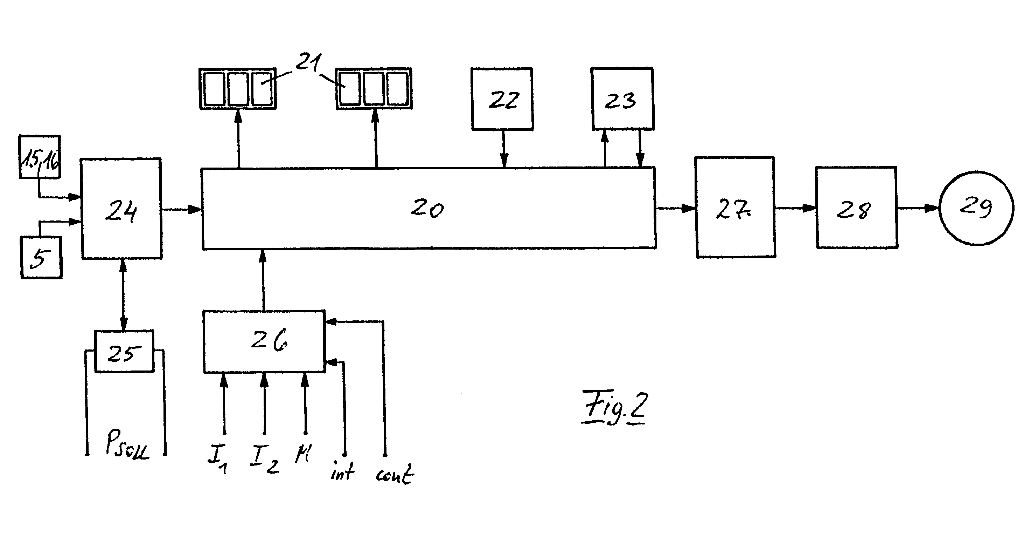

Die in der Fig. 2 als Blockschaltbild näher dargestellte Regel- und Meßeinheit 12 umfaßt eine zentrale Recheneinheit (CPU) 20, an welche LED-Anzeigen 21 für die Anzeige des aktuellen Körperhöhlendruckes $P_{IST}$ bzw. des apparativen Druckes $P_{APP}$ angeschlossen sind. Ferner sind ein Programmspeicher 22 und ein Datenspeicher 23 an die zentrale Recheneinheit 20 angeschlossen. Über einen dreikanaligen A/D-Wandler 24 werden die Werte des Flowsensors 15 bzw. 16 und des Drucksensors (Druckanzeigers) 5 sowie ferner der eingestellte Solldruck $P_{SOLL}$ über ein Potentiometer 25 der Regel- und Meßeinheit 12 eingegeben. Als Flowsensor wird dabei entweder der Gewichtssensor 15 am Vorratsbehälter 3 für die Perfusionsflüssigkeit oder der Drehzahlsensor 16 an der Pumpe 4 verwendet. Über eine digitale Ein- und Ausgabe 26 können festeingestellte Werte I1, I2, M als festeingestellte Leitwerte der Perfusionsleitung 9 zwischen dem Drucksensor 5 und dem medizinischen Instrument 6 sowie des medizinischen Instrumentes 6 selbst eingegeben werden. Ferner können über die digitale Ein- und Ausgabe 26 Steuerungsvorgänge- wie ununterbrochene Perfusion (cont) und unterbrochene Perfusion (int)-eingegeben werden. Die festeingestellten Leitwerte und die vorgegebenen Werte für die unterbrochene oder ununterbrochene Perfusion können mittels an der Gerätevorderseite der Regel- und Meßeinheit 12 angebrachter Drucktasten eingestellt werden. Ferner sind dort die LED-Anzeigen 21 für den Körperhöhlendruck $P_{IST}$ und den apparativen Druck $P_{APP}$ angeordnet. Schließlich sind der Programmspeicher 22 und das Potentiometer 25 für die Sollwerteingabe $P_{SOLL}$ auf der Gerätevorderseite angeordnet.

Die Regel- und Meßeinheit 12 steuert über einen D/A-Wandler 27 und eine Leistungsendstufe 28 den Motor 29 der Pumpe 4.

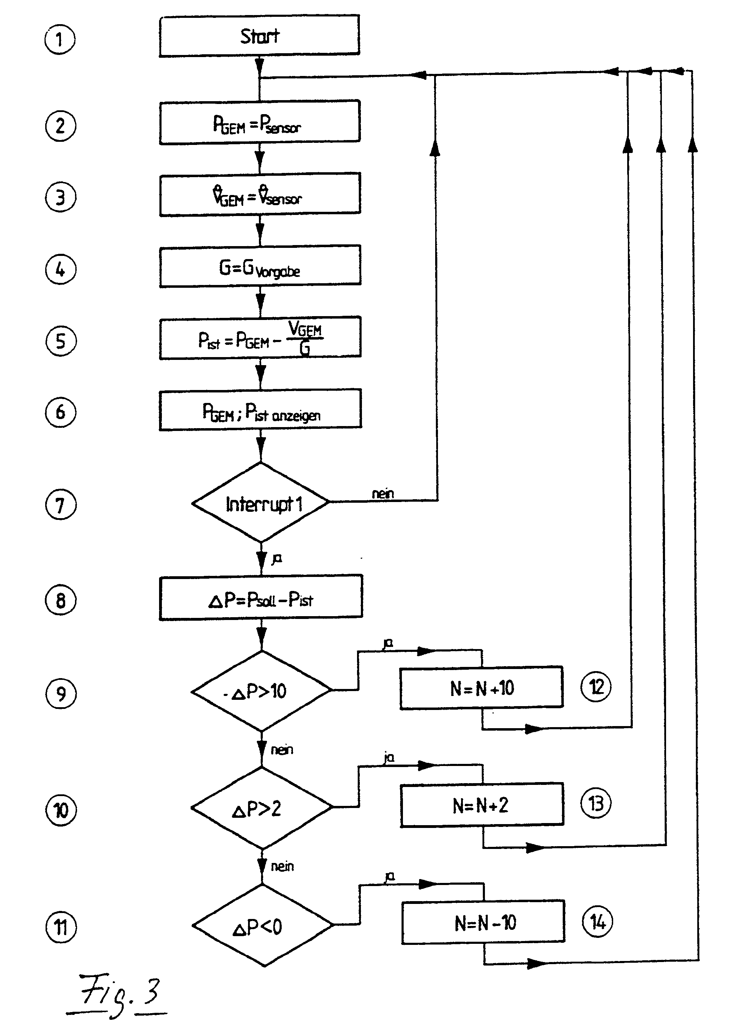

Die beschriebene Vorrichtung arbeitet gemäß dem in Fig. 3 vereinfacht dargestellten Programmablaufplan der Regel- und Meßeinheit 4. Nach dem Auslösen des Startimpulses in Stufe 1 wird in Stufe 2 der vom Drucksensor 5 gemessene Druckwert über den A/D-Wandler 24 gewandelt und der zentralen Recheneinheit 20 eingegeben und als Druckwert $P_{GEM}$ abgespeichert. In Stufe 3 wird der über den Flowsensor 15, 16 gemessene Flowwert $\overset{\circ}{V}_{SENSOR}$ gemessen, vom A/D-Wandler 24 abgewandelt und als Flowwert $\overset{\circ}{V}_{GEM}$ abgespeichert. In Stufe 4 wird der gegen die Atmosphäre ermittelte Leitwert G des verwendeten medizinischen Instrumentes 6 sowie des Schlauchabschnittes 9 ermittelt oder es wird der Leitwert $G_{VORGABE}$ aus der vorher ausgewählten Speicherstelle $I_1$, $I_2$ oder M über die digital Ein- und Ausgabe 26 eingegeben bzw. entnommen. In Stufe 5 wird der aktuelle Körperhöhlendruck $P_{IST}$ aus den vorher ermittelten Werten nach folgender Formel berechnet :

$$P_{IST} = P_{GEM} - \frac{\overset{\circ}{V}_{GEM}}{G}$$

In Stufe 6 werden der errechnete Körperhöhlendruck $P_{IST}$ und der gemessene apparative Druck $P_{GEM}$ an den LED-Anzeigen 20 angezeigt.

In Stufe 7 wird der Taktgebar für die Drucksteuerungs-Auslösung abgefragt. Sofern sich in Stufe 7 das Resultat "nein" ergibt, wird das Programm zur Stufe 2 zurückgeschaltet. Sofern sich das Ergebnis "ja" ergibt wird Stufe 8 erreicht.

In Stufe 8 wird der Differenzdruck $\Delta P$ aus dem Solldruck $P_{SOLL}$ und dem Istdruck $P_{IST}$ nach folgender Formel errechnet :

3

EP 0 329 599 B1

$$\Delta P = P_{SOLL} - P_{IST}$$

Sofern in Stufe 9 sich ein Differenzdruck $\Delta P$ von größer 10 ergibt, wird der Programmablaufplan über die Zwischenstufe 12 zurückgeschaltet zur Stufe 2. Hierbei wird die Drehzahl N der Pumpe 4 erhöht auf N = N + 10. Sofern der Differenzdruck $\Delta P$ kleiner ist als 10 aber noch > 2 wird in Stufe 10 über die Zwischenstufe 13 auf Stufe 2 zurückgeschaltet, wobei die Drehzahl N der Pumpe 4 erhöht wird auf N = N + 2. Sofern der Differenzdruck $\Delta P$ < 2 ist, wird zur Stufe 11 geschaltet, wobei die Drehzahl N der Pumpe 4 erniedrigt wird auf N = N – 10. Der Programmablauf wird über die Zwischenstufe 14 zurückgeschaltet zur Stufe 2. In den Zwischenstufen 12 bis 14 wird somit durch Erhöhung oder Verringerung der Drehzahl der Pumpe 4 eine Erhöhung oder Verringerung des Volumenstromes $\overset{\circ}{V}$ bewirkt, wodurch der Druck in der Körperhöhle 1 mittels kontinuierlicher Regelung der Förderleistung der Pumpe 4 unter Berücksichtigung des eingestellten oder errechneten Leitwertes auf einen voreingestellten Wert konstant gehalten wird.

## Patentansprüche

1. Vorrichtung zur Perfusion von Körperhöhlen mit einer von einem Vorratsbehälter über eine Perfusionsleitung und ein medizinisches Instrument zugeführten Flüssigkeit, mit einer an die Perfusionsleitung angeschlossenen Pumpe, mit einem zwischen der Pumpe und dem medizinischen Instrument an die Perfusionsleitung angeschlossenen Druckaufnehmer, mit einer Einrichtung zur Messung des Volumenstromes und mit einer an den Druckaufnehmer und die Pumpe angeschlossenen Regel- und Meßelektronik, die den aktuellen Druck in der Körperhöhle durch Auswertung des vom Druckaufnehmer gemessenen Druckes ermittelt, **dadurch gekennzeichnet,**
daß die Regel- und Meßelektronik (12) zur Ermittlung des aktuellen Druckes ($P_{IST}$) in der Körperhöhle (1) durch Auswertung des vom Druckaufnehmer (5) gemessenen Druckes ($P_{GEM}$), des über die Pumpe (4) zugeführten Volumenstromes (V) und des Leitwertes ($G_1$) der Perfusionsleitung (9) zwischen dem Druckaufnehmer (5) und dem Instrument (6) sowie des Leitwertes ($G_2$) des Instrumentes (6) vorgesehen ist,
daß eine Einrichtung zur Ermittlung der Leitwerte ($G_1$) der Perfusionsleitung (9) zwischen dem Druckaufnehmer (5) und dem medizinischen Instrument (6) und der Leitwerte ($G_2$) des Instrumentes (6) durch Auslösen einer kurzzeitigen Probeförderung der Flüssigkeit mittels der Pumpe (4) durch das medizinische Instrument (6) gegen die Atmosphäre für verschiedene Volumenströme (V) vorgesehen ist,
daß eine Einrichtung zur Abspeicherung der ermittelten Leitwerte ($G_1$, $G_2$) in der Regel-und Meßelektronik (12) vorgesehen ist und
daß eine Einrichtung zur Konstanthaltung des Druckes ($P_{IST}$) in der Körperhöhle (1) durch kontinuierliche Regelung der Förderleistung der Pumpe (4) unter Berücksichtigung der Leitwerte ($G_1$, $G_2$) auf einen voreingestellten Wert ($P_{SOLL}$) vorgesehen ist.

2. Vorrichtung nach Anspruch 1, dadurch gekennzeichnet, daß die Leitwerte verschiedener Instrumente (6) in der Regel- und Meßelektronik (12) vorab abspeicherbar sind.

3. Vorrichtung nach Anspruch 1 oder 2, dadurch gekennzeichnet, daß ein Gewichtssensor (15) am Vorratsbehälter (3) angeordnet und mit der Regel — und Meßelektronik (12) zur Ermittlung des Volumenstromes (V) verbunden ist.

4. Vorrichtung nach Anspruch 1, dadurch gekennzeichnet, daß ein Drehzahlsensor (16) an der Pumpe (4) angeordnet und mit der Regel- und Meßelektronik (12) zur Ermittlung des Volumenstromes (V) verbunden ist.

5. Vorrichtung nach Anspruch 1, 3 oder 4, dadurch gekennzeichnet, daß ein Volumenstromsensor am Perfusionsschlauch (10) angeordnet und mit der Regel- und Meßelektronik (12) zur Ermittlung des Volumenstromes (V) um Null verbunden ist.

6. Vorrichtung nach einem der Ansprüche 3 bis 5, dadurch gekennzeichnet, daß eine Einrichtung zur kurzzeitigen Stillsetzung der Pumpe (4) mittels der Regel- und Meßelektronik (12) vorgesehen ist, wobei in diesem Zustand der dem aktuellen Körperhöhlendruck entsprechende statische Druck ($P_{IST}$) ermittelt und zur Selbstüberprüfung des errechneten Körperhöhlendruckes verwendet wird.

7. Vorrichtung nach einem der Ansprüche 3 bis 6, dadurch gekennzeichnet, daß die Regel- und Meßelektronik (12) mit LED-Anzeigen (21) zur Anzeige des Körperhöhlendruckes ($P_{IST}$) und des apparativen Druckes ($P_{APP}$) versehen ist.

8. Vorrichtung nach einem der vorangegangenen Ansprüche, dadurch gekennzeichnet, daß die Pumpe (4) durch einen Vorratsbehälter (3) mit steuerbarer Druckmanschette ersetzt ist.

9. Vorrichtung nach einem der vorangegangenen Ansprüche, dadurch gekennzeichnet, daß die Pumpe (4) unter Ausnutzung der Schwerkraft durch einen Vorratsbehälter (3) für die Flüssigkeit mit ausgangsseitig angeordnetem, steuerbarem Volumenstromventil ersetzt ist.

4

## Claims

1. A device for the perfusion of body cavities, comprising a liquid supplied from a supply container over a perfusion line and a medical instrument, a pump connected to the perfusion line, a pressure transducer connected between the pump and the medical instrument, a device for measuring the volume flow and an electronic regulating and measuring circuitry connected to the pressure transducer and the pump, said circuitry determining the actual pressure in the body cavity by evaluation of the pressure measured by the pressure transducer,

characterized by that the electronic regulating and measuring circuitry (12) is provided for determining the actual pressure ($P_{ACT}$) in the body cavity (1) by evaluation of the pressure ($P_{MEAS}$) measured by the pressure transducer (5), of the volume flow (V) supplied over the pump (4) and of the conductivity ($G_1$) of the perfusion line (9) between the pressure transducer (5) and the instrument (6), and of the conductivity ($G_2$) of the instrument (6),

that a device is provided for determining the conductivities ($G_1$) of the perfusion line (9) between the pressure transducer (5) and the medical instrument (6), and the conductivities ($G_2$) of the instrument (6) by releasing a short-time sample supply of liquid by means of the pump (4) through the medical instrument (6) against the atmosphere for different volume flows (V),

that a device is provided for storing the determined conductivities ($G_1$, $G_2$) in the electronic regulating and measuring circuitry (12), and

that a device is provided for holding the pressure ($P_{ACT}$) in the body cavity constant by continuous regulation of the supply capacity of the pump (4) under consideration of the conductivities ($G_1$, $G_2$) at a preset value ($P_{DES}$).

2 A device according to claim 1, characterized by that the conductivities of different instruments (6) can be stored beforehand in the electronic regulating and measuring circuitry (12).

3. A device according to claims 1 or 2, characterized by that a weight sensor (15) is attached at the supply container (12) and is connected with the electronic regulating and measuring circuitry (12) for determining the volume flow (V).

4. A device according to claim 1, characterized by that a speed sensor (16) is provided at the pump (4) and is connected with the electronic regulating and measuring circuitry (12) for determining the volume flow (V).

5. A device according to claims 1, 3 or 4, characterized by that a volume flow sensor is provided at the perfusion hose (10) and is connected with the electronic regulating and measuring circuitry (12) for determining the volume flow (V) in the order of zero.

6. A device according to one of claims 3 to 5, characterized by that a device for short-time stopping of the pump (4) by means of the electronic regulating and measuring circuitry (12) is provided, in this condition the static pressure ($P_{ACT}$) corresponding to the actual body cavity pressure being determined and being used for self-monitoring of the calculated body cavity pressure.

7. A device according to one of claims 3 to 6, characterized by that the electronic regulating and measuring circuitry (12) is provided with LED displays (21) for indicating the body cavity pressure ($P_{ACT}$) and the pressure of apparatus ($P_{APP}$).

8. A device according to one of the preceding claims, characterized by that the pump (4) is replaced by a supply container (3) with controllable pressure sleeve.

9. A device according to one of the preceding claims, characterized by that the pump (4) is replaced, under making use of gravity, by a supply container (3) for the liquid with a volume flow valve arranged at the output side thereof.

## Revendications

1. Dispositif pour la perfusion des cavités d'un corps, comprenant un liquide alimenté à partir d'un réservoir par l'intermédiaire d'une ligne de perfusion et d'un instrument médical, une pompe raccordée à la ligne de perfusion, un capteur de pression raccordé entre la pompe et l'instrument médical à la ligne de perfusion, un dispositif pour la mesure du courant volumétrique et une électronique de régulation et de mesure raccordée au capteur de pression et à la pompe, ladite électronique déterminant la pression actuelle dans la cavité d'un corps par l'évaluation de la pression mesurée par le capteur de pression,

caractérisé en ce que l'électronique de régulation et de mesure (12) est prévue pour la détermination de la pression actuelle ($P_{ACT}$) dans la cavité (1) d'un corps par l'évaluation de la pression ($P_{MES}$) mesurée par le capteur de pression (5), du courant volumétrique (V) alimenté par l'intermédiaire de la pompe (4) et de

5

la conductance ($G_1$) de la ligne de perfusion (9) entre le capteur de pression (5) et l'instrument (6) et de la conductance ($G_2$) de l'instrument (6),

qu'un dispositif est prévu pour la détermination des conductances ($G_1$) de la ligne de perfusion (9) entre le capteur de pression (5) et l'instrument médical (6) et des conductances ($G_2$) de l'instrument (6) par relâchement d'une prise d'essai alimentée de courte durée du liquide au moyen de la pompe (4) à travers de l'instrument (6) contre l'atmosphère pour les différents courants volumétriques (V),

qu'un dispositif pour mémoriser la conductance déterminée ($G_1$, $G_2$) dans l'électronique de régulation et de mesure est prévu, et

qu'un dispositif est prévu pour maintenir la pression ($P_{ACT}$) dans la cavité (1) d'un corps par régulation continue de la capacité d'alimentation de la pompe (4), sous considération des conductances ($G_1$, $G_2$), constante à une valeur ($P_{CON}$) préréglée.

2. Dispositif selon la revendication 1, caractérisé en ce que les conductances de différents instruments (6) sont mémorisables auparavant dans l'électronique de régulation et de mesure (12).

3. Dispositif selon la revendication 1 ou 2, caractérisé en ce qu'un capteur de poids (15) est prévu au réservoir (3) et est raccordé à l'électronique de régulation et de mesure (12) pour déterminer le courant volumétrique (V).

4. Dispositif selon la revendication 1, caractérisé en ce qu'un capteur de vitesse rotative (16) est prévu à la pompe (4) et est raccordé à l'électronique de régulation et de mesure (12) pour déterminer le courant volumétrique (V).

5. Dispositif selon les revendications 1, 3 ou 4, caractérisé en ce qu'un capteur de courant volumétrique est prévu au tuyau de perfusion et est raccordé à l'électronique de régulation et de mesure (12) pour déterminer le courant volumétrique (V) dans l'ordre de zéro.

6. Dispositif selon une des revendications 3 à 5, caractérisé en ce qu'un dispositif est prévu pour l'arrêt de courte durée de la pompe (4) au moyen de l'électronique de régulation et de mesure (12), dans cet état la pression statique ($P_{ACT}$) correspondant à la pression actuelle de la cavité d'un corps étant déterminée et utilisée pour l'auto-surveillance de la pression calculée de la cavité d'un corps.

7. Dispositif selon une des revendications 3 à 6, caractérisé en ce que l'électronique de régulation et de mesure (12) est pourvue d'affichages LED (21) pour indiquer la pression ($P_{ACT}$) de la cavité d'un corps et de la pression de l'appareil ($P_{APP}$).

8. Dispositif selon une des revendications précédentes, caractérisé en ce que la pompe (4) est remplacée par un réservoir (3) avec une manchette de pression commandable.

9. Dispositif selon une des revendications précédentes, caractérisé en ce que la pompe (4) est remplacée, sous utilisation de la gravité, par un réservoir (3) pour le liquide avec une soupape de courant volumétrique commandable à la sortie.

Fig. 1

21

15.16

5

24

25

$P_{soll}$

26

$I_1$  $I_2$  $M$  int  cont

22

23

20

27

28

29

8

Fig. 2

EP 0 329 599 B1

① $\boxed{\text{Start}}$

② $\boxed{P_{GEM} = P_{sensor}}$

③ $\boxed{\dot{V}_{GEM} = \dot{V}_{sensor}}$

④ $\boxed{G = G_{vorgabe}}$

⑤ $\boxed{P_{ist} = P_{GEM} - \dfrac{V_{GEM}}{G}}$

⑥ $\boxed{P_{GEM} ; P_{ist} \text{ anzeigen}}$

⑦ $\langle \text{Interrupt 1} \rangle$ nein

ja

⑧ $\boxed{\triangle P = P_{soll} - P_{ist}}$

⑨ $\langle -\triangle P > 10 \rangle$ ja $\boxed{N = N + 10}$ ⑫

nein

⑩ $\langle \triangle P > 2 \rangle$ ja $\boxed{N = N + 2}$ ⑬

nein

⑪ $\langle \triangle P < 0 \rangle$ ja $\boxed{N = N - 10}$ ⑭

Fig. 3